# FASCICULE DE BREVET EUROPEEN

(11) **EP 0 373 012 B1**
(45) Date de publication et mention de la délivrance du brevet: **01.02.1995**
(21) Numéro de dépôt: 89403065.9
(22) Date de dépôt: 07.11.1989
(51) Int. Cl.: C12N 15/57, C12N 5/10, C12N 9/64, A61K 38/46

(54) **ADN codant pour le facteur IX ou protéine analogue**
DNS, die für Faktor IX oder analoges Protein kodiert
DNA encoding for factor IX or an analogous protein

(30) Priorité: 09.11.1988 FR 8814635
(43) Date de publication de la demande: 13.06.1990
(73) Titulaire: TRANSGENE S.A., 67082 Strasbourg Cédex (FR)
(72) Inventeur: Meulien, Pierre, F-67000 Strasbourg (FR)
(74) Mandataire: Warcoin, Jacques

(56) Documents cités:
- WO-A-88/03926
- CELL, vol. 48, 1987, pages 185-191; M.J. JORGENSEN et al.: "Recognition sitedirecting vitamin K-dependent gamma-carboxylation resides on the propeptide offactor IX"
- NUCLEIC ACIDS RESEARCH, vol. 15, no. 22, 25 novembre 1987, pages 9505-9513, IRLPress Ltd, Oxford, GB; P. GALEFFI et al.: "The propeptide region of clottingfactor IX is a signal for a vitamin K dependent carboxylase: evidence from protein engineering of amino acid-4"
- PROC. NATL. ACAD. SCI. USA, vol. 76, no. 10, octobre 1979, pages 4990-4994; K.KATAYAMA et al.: "Comparison of amino acid sequence of bovine coagulationfactor IX (Christmas Factor) with that of other vitamin K-dependent plasmaproteins

## Description

La présente invention concerne une nouvelle séquence d'ADN codant pour le facteur IX humain ou une protéine analogue, utilisable pour son expression dans une cellule hôte, en particulier une cellule de vertébrés, notamment de mammifères.

Le Facteur IX est une protéine dépendante de la vitamine K, qui intervient au cours de la coagulation du sang. Il est synthétisé sous forme d'un zymogène et subit trois types de modifications post-traductionnelles avant d'être secrété dans le sang : la conversion des acides glutamiques en acides carboxyglutamiques, dépendant de la vitamine K, l'addition de chaines hydrocarbonées et la β hydroxylation d'un acide aspartique. C'est le foi qui chez l'homme est le site de synthèse du facteur IX. Cette protéine intervient au cours du cycle de la coagulation du sang et est utilisée pour le traitement des hémophiles B. A l'heure actuelle, la seule source commercialement disponible de facteur IX est le plasma humain.

Toutefois, les préparations de facteur IX obtenues par extraction à partir du plasma peuvent être pyrogènes et comportent également des risques de contamination par des agents pathogènes ou des virus notamment le virus de l'hépatite ou les agents vecteurs du Sida.

C'est pourquoi il est particulièrement intéressant de développer des moyens de préparation du facteur IX avec une très haute pureté et ne mettant pas en jeu l'extraction à partir du plasma humain ou animal.

Depuis quelques années, des tentatives ont donc été faites pour préparer du facteur IX en utilisant les techniques de l'ADN recombinant.

En particulier, la Demanderesse a décrit dans des demandes de brevet antérieures, les publications européenes de brevet EP 0167420, EP 0162782 et EP 0251874, des procédés permettant de transformer des cellules hôtes afin de les rendre capables de produire le Facteur IX.

Parmi toutes les cellules hôtes envisagées, les cellules de mammifères ont donné les résultats les plus intéressants, conformément à ce qu'on pouvait attendre des résultats déjà obtenus pour l'expression d'autres protéines dépendantes de la vitamine K. Par exemple, l'expression du facteur VII ou de la protéine C dans des cellules de mammifères a été un succès, conduisant à l'obtention de protéines parfaitement actives. (Berkner K. et al Cold Spring Harbor: Symposia on quantitative Biology vol LI 1986)

On a toutefois rapidement constaté la spécificité du facteur IX et la difficulté à obtenir un produit à la fois actif et avec un rendement suffisant pour envisager un développement industriel à un coût qui ne soit pas dissuasif.

Une publication récente (Rees DJG et al, Embo J. ; 1988 7, 2053) décrit ainsi l'obtention de facteur IX très actif à partir de cellules de reins de chiens (MDCK), mais le niveau d'expression est extrêmement bas, de l'ordre de 0.2 à 0.3 ug / 10⁶ cellules / 24 heures. On a pu constater qu'à ces faibles niveaux d'expression, la réaction de carboxylation, indispensable pour conférer son activité au facteur IX, n'est pas saturée, ce qui explique la très grande activité spécifique observée.

On connait par ailleurs bien la structure du produit primaire de traduction du cDNA du facteur IX et on peut la décomposer en 3 domaines : à l'extrémité N-terminale se trouve une séquence signal de 28 acides aminés qui est clivée lorsque le produit de traduction primaire traverse la membrane du réticulum endoplasmique. Ensuite se trouve une proséquence de 18 acides aminés qui dirige la carboxylation des 12 acides glutamiques, puis la séquence codant pour le facteur IX mature. Des travaux récents ont démontré que deux éléments étaient essentiels pour conférer au facteur IX son activité biologique : d'une part, la carboxylation dépendante de la vitamine K, d'autre part, le clivage de la proséquence lorsque le facteur IX est secrété hors de la cellule.

La présente invention, compte tenu des données scientifiques récentes sur les mécanismes de maturation du facteur IX dans les cellules du foie, vise donc à résoudre les problèmes rencontrés dans les précédentes tentatives de préparation du facteur IX par génie génétique et en particulier, à fournir des moyens de préparer, à un niveau d'expression compatible avec une exploitation industrielle, du facteur IX humain, ou une protéine analogue qui soit biologiquement actif.

La présente invention concerne un analogue du facteur IX humain présentant une homologie substantielle avec le facteur IX humain et ayant le même type d'activité biologique caractérisé en ce qu'il présente une alanine à l'extrémité N-terminale en remplacement de la tyrosine qui se trouve normalement à l'extrémité N-terminale du facteur IX humain.

D'une manière préférentielle, l'analogue du facteur IX humain est l'analogue [Ala]₁ facteur IX humain.

La présente invention concerne également un fragment d'ADN qui comprend au moins une portion codant pour un analogue du facteur IX humain défini ci-dessus.

Le fragment d'ADN comprend au moins :
- une première portion codant pour l'analogue du facteur IX humain;
- une portion située à l'extrémité 5' de ladite première portion et codant pour une proséquence qui en position -3 présente une valine, une arginine, une lysine, une théonine ou une sérine.

De préférence, il comprend une troisième portion, à l'extrémité 5' de ladite seconde portion, laquelle code pour un peptide signal.

L'invention a notamment pour objet un fragment d'ADN qui comprend au moins :
- une première portion codant pour un analogue du facteur IX humain selon la revendication 1 ; et
- une seconde portion située à l'extrémité 5' de ladite première portion et qui code pour une proséquence, laquelle proséquence est dérivée de la proséquence du facteur IX humain, dans laquelle la proline en position -3 est remplacée par une valine, une arginine, une lysine, une thréonine ou une sérine.

Ce fragment d'ADN comprend en outre une troisième portion, située à l'extrémité 5' de ladite seconde portion et qui code pour le peptide signal du précurseur du facteur IX humain.

Dans la suite de la description, les codons numérotés négativement correspondent à la proséquence et le premier codon pour un acide aminé du facteur IX mature est désigné par +1.

Ainsi, de préférence, la séquence d'ADN comporte une séquence codant pour : 
- X - Lys - Arg - Ala -
(-3) (-2) (-1) (+1)
où X est choisi parmi Val, Arg, Lys, Thr, Ser.

De façon générale, on entendra désigner par "protéine analogue au facteur IX humain" dans la description une protéine de structure analogue tout en ayant le même type d'activité biologique in vivo, c'est à dire par exemple, une protéine ayant la même structure primaire que le facteur IX avec éventuellement quelques modifications au niveau d'un nombre réduit d'acides aminés, ou encore une protéine ne présentant pas exactement les mêmes modifications post-traductionnelles que le facteur IX ou bien le même type de protéine comportant des délétions dans les parties non essentielles.

Le produit primaire de traduction du cDNA du facteur IX comporte en fait 3 domaines comme indiqué précédemment. La séquence d'ADN selon l'invention comporte de préférence également une partie codant pour une séquence signal dont la présence peut favoriser l'expression du facteur IX. Il n'est toutefois pas indispensable que cette partie corresponde à la séquence signal naturelle du facteur IX. On peut éventuellement la remplacer par la séquence signal d'autres protéines vitamine K dépendantes, comme le facteur VII ou la protéine C.

Le procédé permettant la préparation d'un clone porteur du gène du facteur IX ne sera pas redécrit en détail ; on pourra se reporter aux publications de brevets déjà mentionnées, dont le contenu est ici incorporé par référence.

Parmi les vecteurs utilisables, on peut citer les virus de la famille des Poxvirus, en particulier le virus de la vaccine ou virus de Cowpox, de préférence le virus de la vaccine, ou encore des vecteurs plasmidiques, en particulier des vecteurs intégratifs. Les différentes alternatives ont été décrites en détail dans 10 des demandes de brevet citées, en particulier EP-0 162 782 et EP-0 251 874.

Les cellules hôtes peuvent être variées tout en étant adaptées au vecteur mis en oeuvre, notamment suivant qu'il s'agit d'un vecteur viral ou d'un vecteur plasmidique. On utilise plus particulièrement les cellules de mammifères, et notamment les cellules de reins telles que les cellules VERO ou les cellules BHK 21. Pour infecter les cellules CHO, on utilise un vecteur dérivé du virus du Cowpox.

Les cellules sont cultivées sur un milieu approprié assurant leur croissance. Après transformation ou infection, les cellules ou les milieux de culture sont récoltés et la protéine du facteur IX ou son analogue actif peut être isolée par des techniques connues de purification des protéines.

Dans la plupart des cas, il est important que la mise en culture se fasse sur un milieu comportant de la vitamine K, dont la quantité peut varier en fonction des cultures cellulaires, mais sera de préférence en quantité assurant la saturation du milieu, par exemple entre 5 et 50 ug/ml de milieu de culture et en général sera de l'ordre de 10 ug/ml.

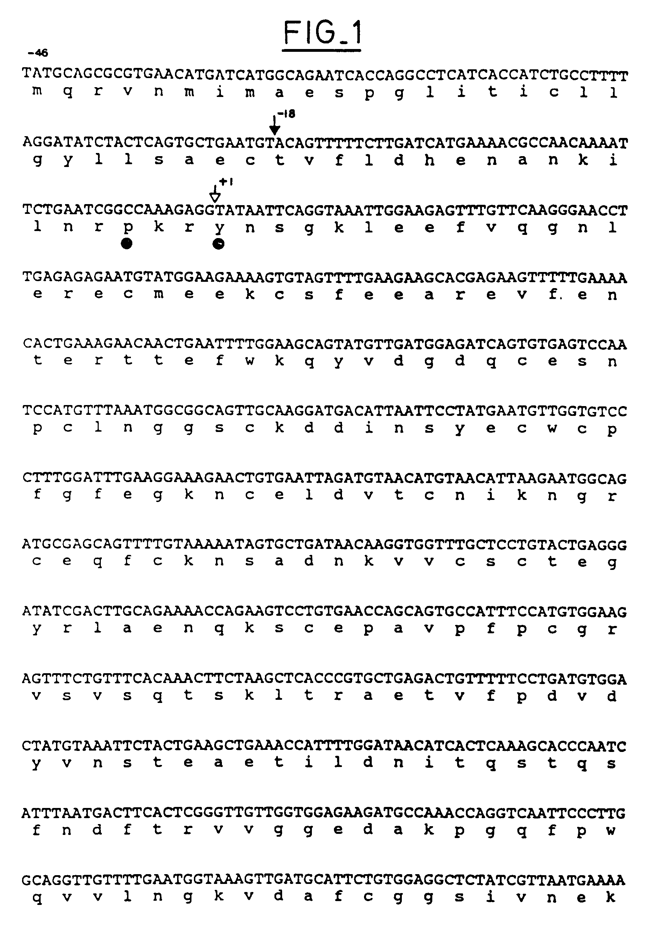

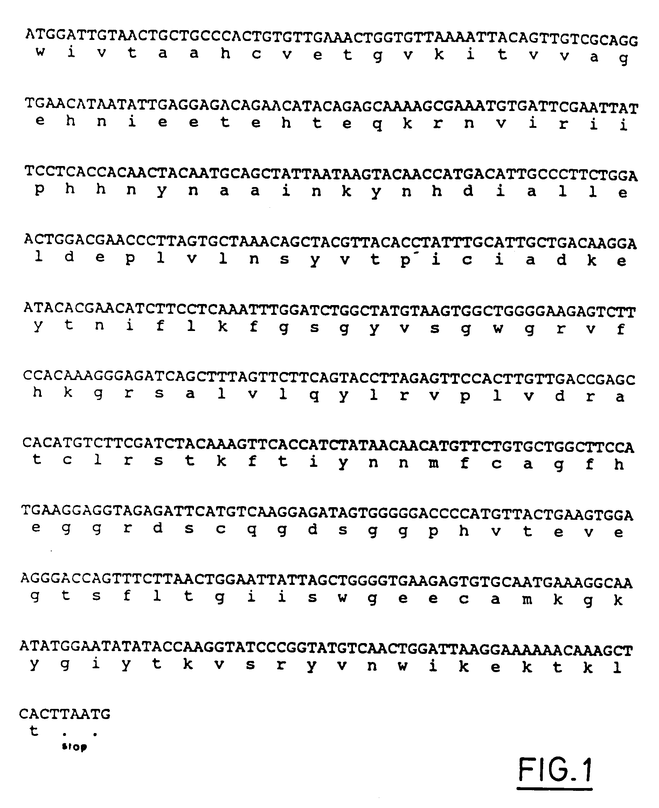

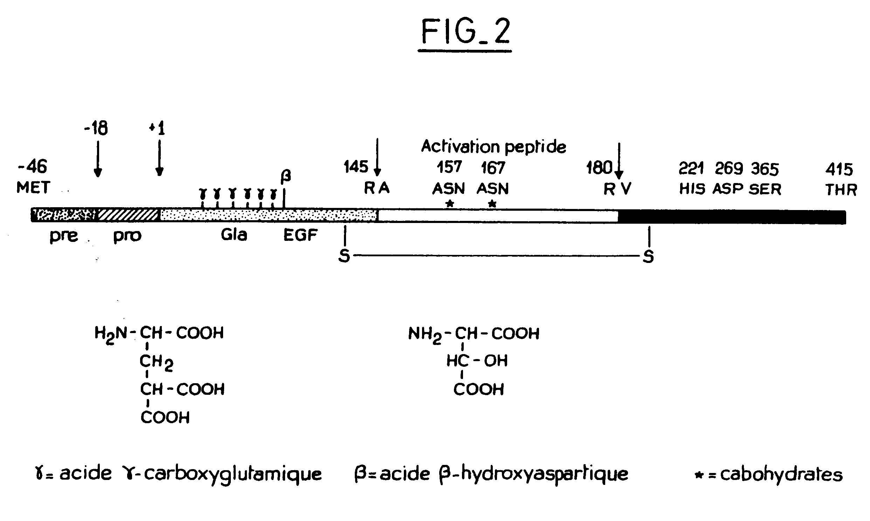

D'autres caractéristiques et avantages de l'invention apparaîtront au cours de la description ci-après, illustrée par les figures 1 et 2, parmi lesquelles :
- la figure 1 représente la séquence de nucléotides du Facteur IX humain et la séquence d'acides aminés correspondante
- la figure 2 représente schématiquement les différents domaines de la structure du produit primaire de traduction du cDNA de la figure 1.

### Exemple 1 : Construction des cDNA du Facteur IX

Le cDNA du Facteur IX a été cloné sous forme d'un fragment Bam HI dans le plasmide pTG 381. La construction pTG 381 a été réalisée pour rendre plus conforme l'extrémité 5′ non codante aux règles de Kozak. La construction du plasmide pTG 381 est indiquée dans la publication de brevet EP-A-0251874 déjà citée. Le fragment Bam HI est recloné dans le phage M13TG127, qui est identique au phage M13TG131 décrit dans Kieny et al 1983 gene, sauf que les sites de restriction dans le polylinker sont Bgl II, Pst I, Hind III, SmaI, Bam HI et EcoRI. La nouvelle construction dans laquelle l'extrémité 5′ du cDNA du Facteur IX est adjacente au site Sma I est appelée M13TG1938.

A partir du phage M13TG1938, on prépare l'ADN simple brin et on fait les mutations sur l'ADNc du Facteur IX en utilisant la technique bien connue de mutagenèse par site dirigé (Zoller et Smith).
a) construction de l'ADNc correspondant à une séquence d'acides aminés dans laquelle la proline (-3) est changée en valine.
   On utilise un oligonucléotide de synthèse désigné par TG1771 : 5′-ATACTCCTCACCCGATTCAG-3′.

Cet oligonucléotide est rapidement hybridé au fragment d'ADN simple brin à 50°C pendant 30 minutes. On prépare alors la molécule double brin en traitant le mélange hybridé avec le fragment Klenow de polymérase en présence de déoxynucléotide triphosphate. Après transformation, les molécules ayant effectivement subi les mutations souhaitées sont identifiées par hybridation en utilisant comme sonde l'oligonucléotide de TG1771 marqué radioactivement.

Pour vérifier que les mutations souhaitées ont bien été introduites, on détermine la séquence d'ADN de l'extrémité 5′ du nouveau cDNA du Facteur IX. Le codon CCA codant par la proline est remplacé par le codon GTG codant pour la valine.
b) Construction de l'ADNc correspondant à une séquence d'acides aminés dans laquelle la tyrosine (+1) est changée en alanine.
   On utilise un oligonucléotide de synthèse désigné par TG1770 : 5′-CTGAATTAGCCCTCTTTACCCGATTC-3′.
   Dans ce cas, le codon TAT codant pour la tyrosine est remplacé par le codon GCC codant pour l'alanine. Les conditions sont identiques à celle décrites en a).
c) Construction de l'ADNc correspondant à une séquence d'acides aminés dans laquelle la tyrosine (+1) est changée en alanine et la proline (-3) est changée en valine.
   On utilise un oligonuclétotide de synthèse désigné par TG1327 : 5′-CTGAATTAGCCCTCTTACCCGATTC-3′.
   Le codon CCA codant pour la proline (-3) est remplacé par le codon GTA codant pour la valine, et le codon TAT codant pour la tyrosine (+1) est remplacé par le codon GCT codant pour l'alanine.

### Exemple 2 : obtention des virus de la vaccine recombinants et infection des cellules BHK21

Les nouveaux cDNA ainsi préparés sont excisés du vecteur M13 en utilisant l'enzyme de restriction Bam HI, les fragments sont isolés par électrophorèse sur gel d'd'agarose et insérés dans le vecteur plasmidique pTG186 poly, digéré par Bam HI. Le pTG186 poly est un dérivé du pTG186, dont la construction est décrite dans la publication de brevet EP0162782 déjà citée, qui contient en outre le fragment BglII-EcoRI du polylinker du phage M13TG131 déjà cité.

Les trois plasmides obtenus :
pTG 3533 correspondant à FIX - 3 val/+1 ala (oligonucléotide TG 1327)
pTG 3536 correspondant à FIX - 3 val (oligonulcéotide TG 1771)
pTG 3537 correspondant à FIX +1 ala (oligonucléotide TG 1770)
sont respectivement insérés dans le génome du virus de la vaccine, dans les conditions décrites dans la publication de brevet EP-0162782.

Les virus de la vaccine recombinants,où les séquences codant pour le facteur IX sont sous le contrôle du promoteur 7.5 K de la vaccine, sont sélectionnés pour le phénotype TK⁻ en utilisant le 5 bromo deoxyuridine.

Les cellules sont mises en culture dans le milieu MEM Glasgow supplémenté avec 5 % de sérum de veau foetal inactivé par chauffage à 56°C pendant 30 minutes, en présence de 10 ug/ml de vitamine K. L'infection avec les virus recombinants est effectuée à un taux d'infection de 1 pfu. Les cellules sont lavées 3 fois avec le milieu seul puis mises en présence de milieu complet. 24 heures après, on teste dans les surnageants de culture la présence du facteur IX de deux façons :
- la quantité d'antigène Facteur IX est estimée avec un test Elisa (commercialisé par Diagnostica Stago).
- l'activité du Facteur IX est calculée en mesurant le temps de coagulation d'un plasma d'hémophile supplémenté avec les extraits (kit commercialisé par Stago).

Les résultats sont fournis dans le tableau joint. La quantité d'antigène Facteur IX est exprimée en ug par ml d'échantillon.

L'activité est exprimée sous forme d'activité spécifique. Pour cela la mesure de l'activité est comparée à celle obtenue en utilisant un mélange de plasmas humain normaux.

L'exemple comparatif indiqué dans le tableau est le virus recombinant VVTG CII dont la construction a été décrite dans la publication de brevet EP-A-0162782, pour l'expression du cDNA du Facteur IX.

On constate que pour un niveau d'expression comparable, l'activité spécifique est fortement augmentée, en particulier avec les virus de la vaccine recombinants VVTG 3533 et VVTG 3537. Le virus VVTG 3537 est particulièrement avantageux puisque l'activité du Facteur IX produit est pratiquement 100 %. Le témoin est un plasma normal, actif à 100 % pour 5 ug/ml de FIX.

Les souches suivantes ont été déposées le 8 novembre 1988 à la Collection Nationale de Cultures de Microorganismes de l'Institut Pasteur (Paris) :

| | |
|---|---|
| - E. coli pTG3537 | sous le n° I-810 |
| - E. coli pTG3536 | sous le n° I-811 |
| - E. coli pTG3533 | sous le n° I-812 |

**TABLEAU**

| **QUANTITE DE FACTEUR IX DETECTE DANS LES CELLULES BHK 21 INFECTEES PAR LES VIRUS DE LA VACCINE RECOMBINANTS** | | | |
|---|---|---|---|
| Virus de la vaccine recombinant | FIX Ag (ug/ml) | activité spécifique (%) FIX activité / FIX Ag | Molécule obtenue |
| VV TG C II | 2.5 | 45 - 55 | F IX naturel |
| VV TG 3536 | 2.0 | 50 - 55 | F IX naturel |
| VV TG 3533 | 1.9 | 70-85 | (Ala¹) FIX |
| VV TG 3537 | 2.3 | 80-100 | (Ala¹) FIX |

## Revendications

1. Analogue du facteur IX humain présentant une homologie substantielle avec le facteur IX humain et ayant le même type d'activité biologique caractérisé en ce qu'il présente une alanine à l'extrémité N-terminale en remplacement de la tyrosine qui se trouve normalement à l'extrémité N-terminale du facteur IX humain.

2. Analogue du facteur IX humain selon la revendication 1 qui est l'analogue [Ala]₁ Facteur IX humain.

3. Un fragment d'ADN qui comprend au moins une portion codant pour un analogue du facteur IX humain selon l'une des revendications 1 et 2.

4. Un fragment d'ADN selon la revendication 3 qui comprend au moins :
- une première portion codant pour l'analogue du facteur IX humain;
- une portion située à l'extrémité 5' de ladite première portion et codant pour une proséquence qui en position -3 présente une valine, une arginine, une lysine, une thréonine ou une sérine.

5. Un fragment d'ADN selon la revendication 4, caractérisé en ce qu'il comprend une troisième portion, à l'extrémité 5' de ladite seconde portion, laquelle code pour un peptide signal.

6. Un fragment d'ADN selon la revendication 3, qui comprend au moins :
- une première portion codant pour un analogue du facteur IX humain selon la revendication 1 ; et
- une seconde portion située à l'extrémité 5' de ladite première portion et qui code pour une proséquence, laquelle proséquence est dérivée de la proséquence du facteur IX humain, dans laquelle la proline en position -3 est remplacée par une valine, une arginine, une lysine, une thréonine ou une sérine.

7. Fragment d'ADN selon la revendication 6 qui comprend en outre une troisème portion, située à l'extrémité 5' de ladite seconde portion et qui code pour le peptide signal du précurseur du facteur IX humain.

8. Un plasmide qui comprend un fragment d'ADN selon l'une des revendications 3 à 7 et des éléments assurant l'expression dudit fragment d'ADN.

9. Vecteur viral dans le génôme duquel est inséré un fragment d'ADN selon l'une des revendications 3 à 7, ainsi que les éléments assurant l'expression dudit fragment d'ADN.

10. Vecteur viral selon la revendication 9, caractérisé en ce qu'il s'agit d'un virus de la vaccine.

11. Une cellule qui est transformée avec un plasmide selon la revendication 8.

12. Une cellule dans le génôme duquel est inséré un fragment d'ADN selon l'une des revendications 3 à 7 et les éléments assurant l'expression dudit fragment d'ADN dans ladite cellule.

13. Culture cellulaire qui est infectée avec un vecteur viral selon la revendication 10.

14. Un procédé pour préparer un analogue du facteur IX humain selon l'une des revendications 1 et 2, caractérisé en ce qu'on cultive une cellule selon l'une des revendications 11 à 13 et en ce que l'on récupère ledit analogue du facteur IX, à partir de ladite culture.

## Claims

1. Human factor IX analogue displaying substantial homology with human factor IX and having the same type of biological activity, characterized in that it has an alanine at the N-terminal end replacing the tyrosine which normally occurs at the N-terminal end of human factor IX.

2. Human factor IX analogue according to Claim 1, which is the analogue [Ala¹]-human factor IX.

3. A DNA fragment which comprises at least one portion coding for a human factor IX analogue according to either of Claims 1 and 2.

4. A DNA fragment according to Claim 3, which comprises at least:
- a first portion coding for the human factor IX analogue;
- a portion located at the 5' end of the said first portion and coding for a prosequence which has a valine, an arginine, a lysine, a threonine or a serine at position -3.

5. A DNA fragment according to Claim 4, characterized in that it comprises a third portion, at the 5' end of the said second portion, which codes for a signal peptide.

6. A DNA fragment according to Claim 3, which comprises at least:
- a first portion coding for a human factor IX analogue according to Claim 1; and
- a second portion located at the 5' end of the said first portion and which codes for a prosequence, which prosequence is derived from the human factor IX prosequence, in which the proline at position -3 is replaced by a valine, an arginine, a lysine, a threonine or a serine.

7. DNA fragment according to Claim 6, which comprises, in addition, a third portion, located at the 5' end of the said second portion and which codes for the signal peptide of the human factor IX precursor.

8. A plasmid which comprises a DNA fragment according to one of Claims 3 to 7 and elements providing for the expression of the said DNA fragment.

9. Viral vector into the genome of which is inserted a DNA fragment according to one of Claims 3 to 7, as well as the elements providing for the expression of the said DNA fragment.

10. Viral vector according to Claim 9, characterized in that it is a vaccinia virus.

11. A cell which is transformed with a plasmid according to Claim 8.

12. A cell into the genome of which is inserted a DNA fragment according to one of Claims 3 to 7, and the elements providing for the expression of the said DNA fragment in the said cell.

13. Cell culture which is infected with a viral vector according to Claim 10.

14. A method for preparing a human factor IX analogue according to either of Claims 1 and 2, characterized in that a cell according to one of Claims 11 to 13 is cultured, and in that the said factor IX analogue is recovered from the said culture.

## Patentansprüche

1. Analoges des Human-Faktors IX, das eine substantielle Homologie mit dem Human-Faktor IX aufweist und den gleichen Typ von biologischer Aktivität besitzt, dadurch gekennzeichnet, daß es ein Alanin am N-terminalen Ende aufweist, indem das Tyrosin, das sich normalerweise am N-terminalen Ende des Human-Faktors IX befindet, ausgetauscht ist.

2. Analoges des Human-Faktors IX nach Anspruch 1, das das Analoge [Ala]₁-Human-Faktor IX ist.

3. DNA-Fragment, das mindestens einen für ein Analoges des Human-Faktors IX nach einem der Ansprüch 1 und 2 codierenden Teil umfaßt.

4. DNA-Fragment nach Anspruch 3, das mindestens umfaßt:
- einen ersten Teil, der für das Analoge des Human-Faktors IX codiert;
- einen Teil, der sich am Ende 5' des genannten ersten Teiles befindet und für eine Prosequenz codiert, die in Position -3 ein Valin, Arginin, Lysin, Threonin oder Serin aufweist.

5. DNA-Fragment nach Anspruch 4, dadurch gekennzeichnet, daß es einen dritten Teil am Ende 5' des genannten zweiten Teiles umfaßt, der für ein Signalpeptid codiert.

6. DNA-Fragment nach Anspruch 3, das mindestens umfaßt:
- einen ersten Teil, der für ein Analoges des Human-Faktors IX nach Anspruch 1 codiert; und
- einen zweiten Teil, der sich am Ende 5' des genannten ersten Teiles befindet und für eine Prosequenz codiert, wobei die Prosequenz von der Prosequenz des Human-Faktors IX abgeleitet ist, in der das Prolin in Position -3 durch ein Valin, Arginin, Lysin, Threonin oder Serin ersetzt ist.

7. DNA-Fragment nach Anspruch 6, das außerdem einen dritten Teil umfaßt, der sich am Ende 5' des genannten zweiten Teiles befindet und der für das Signalpeptid des Vorläufers des Human-Faktors IX codiert.

8. Plasmid, das ein DNA-Fragment nach einem der Ansprüche 3 bis 7 und Elemente umfaßt, die die Expression des genannten DNA-Fragmentes gewährleisten.

9. Viraler Vektor in dem Genom, von dem ein DNA-Fragment nach einem der Ansprüche 3 bis 7 sowie die Elemente eingefügt werden, die die Expression des genannten DNA-Fragmentes gewährleisten.

10. Viraler Vektor nach Anspruch 9, dadurch gekennzeichnet, daß es sich um einen Virus von Vaccinia handelt.

11. Zelle, die mit einem Plasmid nach Anspruch 8 transformiert ist.

12. Zelle in dem Genom, von dem ein DNA-Fragment nach einem der Ansprüche 3 bis 7 sowie die Elemente eingefügt werden, die die Expression des genannten DNA-Fragmentes in die genannte Zelle gewährleisten.

13. Zellkultur, die mit einem viralen Vektor nach Anspruch 10 infiziert wird.

14. Verfahren zur Herstellung eines Analogen des Human-Faktors IX nach einem der Ansprüche 1 und 2, dadurch gekennzeichnet, daß man eine Zelle nach einem der Ansprüche 11 bis 13 kultiviert und dadurch, daß man das genannte Analoge des Faktors IX ausgehend von dieser Kultur gewinnt.
